# EUROPEAN PATENT SPECIFICATION

(11) **EP 0 519 600 B1**
(45) Date of publication and mention of the grant of the patent: **11.06.1997**
(21) Application number: 92304314.5
(22) Date of filing: 13.05.1992
(51) Int. Cl.: C07D 471/10, C07D 217/26

(54) **Process and intermediate for making isoquinoline derivatives**
Prozess und Zwischenprodukte bei der Darstellung von Isoquinolinderivaten
Procédé et intermédiaires de fabrication de dérivés d'isoquinoline

(30) Priority: 22.05.1991 US 703969; 08.04.1992 GB 9207634
(43) Date of publication of application: 23.12.1992
(73) Proprietor: AMERICAN HOME PRODUCTS CORPORATION, Madison, New Jersey 07940-0874 (US)
(72) Inventor: Malamas, Michael Sotirios, Jamison, Pennsylvania 18929 (US)
(74) Representative: Connelly, Michael John

(56) References cited:
- EP-A- 254 149
- EP-A- 365 324
- US-A- 5 106 978
- ANNALES DE CHIMIE-SCIENCE DES MATERIAUX, vol. 5, no. 1, 1970, Paris, FR; P. L. COMPAGNON, M. MIOQUE; "Addition des reactifs nucleophiles sur la triple liaison nitrile", page 16.

## Description

The present invention relates to a process for the preparation of spiro[isoquinoline-4(1H),3'-pyrrolidine]-1,2',3,5'(2H)-tetrones which are useful as aldose reductase inhibitors. The invention also relates to novel intermediates useful in the process.

EP 0 365 324A discloses a class of nitriles having the general formula I 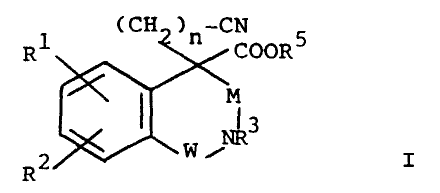 in which R¹ and R² represent hydrogen or various substituents, R³ represents hydrogen or various substituents, M and W are independently carbonyl, thiocarbonyl, sulfonyl, sulfoxo or C₁ or C₂ lower alkylene and n is 1 to 3. The nitriles are disclosed for the purpose of preparing thioamides by reaction with HSP(=S)(OEt)₂/HCl. The present invention includes a selected novel subgenus of compounds falling within the said class. The novel compounds have the advantage of being useful in an improved method of preparing spiro[isoquinoline-4(1H),3'-pyrrolidine]-1,2',3,5'(2H)-tetrones from 1,2,3,4-tetrahydro-1,3-dioxo-4-isoquinolinecarboxylic acid esters.

EP 0 365 324A also discloses a method of preparing spiro [isoquinoline-4(1H),3'-pyrrolidine]-1,2',3,5' (2H)-tetrones from 1,2,3,4-tetrahydro-1,3-dioxo-4-isoquinolinecarboxylic acid esters. The method comprises 4 steps. The starting compound is reacted with t-butyl bromacetate in the presence of K₂CO₃ to introduce CH₂COO^{t}Bu at the 4-position. The second step comprises removal of the tertiary butyl group with trifluoroacetic acid to produce the 4-(carboxymethyl) derivative of the starting compound. The third step comprises reacting the carboxylic acid with a coupling agent followed by ammonia to prepare the 4-(2-amino-2-oxoethyl) derivative of the starting compound. The fourth step comprises cyclization of this amide in the presence of a suitable base.

The prior art method employs three steps for the conversion of the starting compound into its 4-(2-amino-2-oxoethyl) derivative. The present invention provides a process in which this conversion is carried out in fewer steps, namely two steps. Each of these steps can be performed in very high yield. Thus the invention has the advantage that it can be carried out in higher yields than the prior art method.

EP-A-0 254 149 teaches that 2',3'-dihydrospiro [pyrroline-3,6'(5'H)-pyrrolo[1,2,3-de][1,4]benzoxazine]-2,5,5'-triones can be prepared from 6-cyanomethyl-2,3-dihydro-5-oxopyrrolo[1,2,3-de]-1,4-benzoxazine-6-carboxylic acid lower alkyl esters either directly or via 6-carbamoylmethyl-2,3-dihydro-5-oxopyrrolo[1,2,3-de]-1,4-benzoxazine-6-carboxylic acid lower alkyl esters.

The present invention provides a process for the preparation of a compound having the formula II 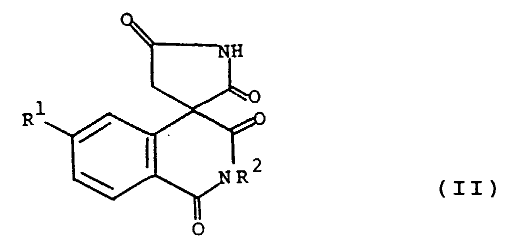 in which R¹ is hydrogen or halogen and R² is methyl or dihalogen- substituted benzyl, in which
(1) a compound having the formula III 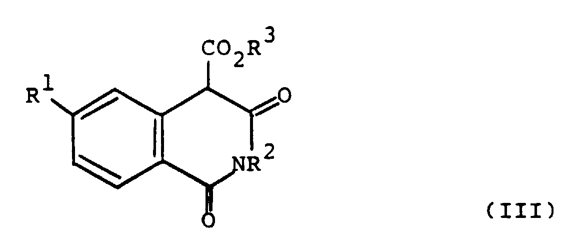 in which R¹ and R² are as defined above and -CO₂R³ is an ester group preferably -CO₂Me, is cyanomethylated to prepare a compound having the formula IV 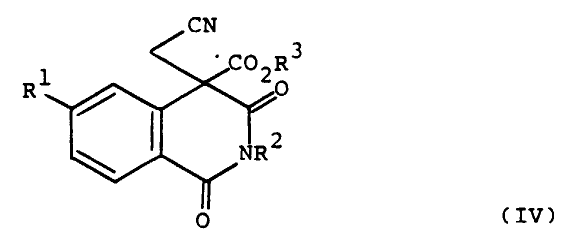 in which R¹, R² and -CO₂R³ are as defined above;
(2) the compound having formula IV is hydrated to prepare an amide having the formula V 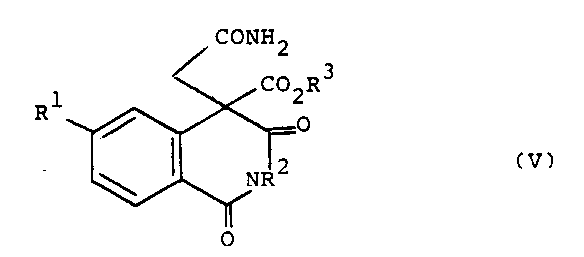 in which R¹, R² and CO₂R³ are as defined above; and
(3) the amide having the formula V is cyclized with a base to form the compound having formula II. The invention also provides the nitriles having formula IV as new compounds.

R¹ is preferably halogen, more preferably fluorine. R² is preferably dihalogen- substituted benzyl, more preferably (4-bromo-2-fluorophenyl) methyl. R³ is preferably methyl.

The compounds having formula III can be prepared in manner known **per se**. Our preferred compounds of formula III, the methyl esters, are preferably prepared by the steps designated (a) and (b) below.

The cyanomethylation reaction can be carried out by reaction of the compound having formula III with a cyanomethylating agent, i.e. a compound having the formula CNCH₂X where X is a leaving group, preferably bromine, in the presence of a base such as potassium carbonate. The reaction can be performed in a conventional solvent. We prefer to carry out the reaction at about 0°C in a a combination of DMF and acetone. The cyanomethylation can be carried out in yields of around 94% and 95%.

The conversion of the nitrile (IV) into the amide (V) is preferably carried out in a manner avoiding aqueous base or aqueous acid in order to avoid hydrolysis of the ester group -CO₂R³. Thus the hydration of the nitrile (IV) is suitably performed under non-hydrolytic conditions. We prefer to carry out the hydration by reaction of the nitrile with an alcohol and a hydrogen halide (hydrogen chloride or hydrogen bromide) under essentially anhydrous conditions. The reaction is preferably carried out in the presence of diethyl ether as solvent. Excess of the alcohol may also serve as additional solvent. As preferred reactants one may use by hydrogen chloride gas and methanol. These reactants are believed to produce an imido ester salt which readily decomposes to form methyl chloride and the desired amide (V). The conversion of the nitrile (IV) into the amide (V) can be carried out in yields of about 92% to 95%.

The cyclization of the amide (V) is preferably carried out in the presence of NaH in DMF.

If R3 in formula III is homochiral then the intermediate compounds of formulae IV and V will be a mixture of diastereoisomers which may be separated by means known in the art, e.g crystallisation and chromotography. In this case cyclization of compound V will give a single enantiomer of formula II.

The process of the present invention is illustrated by the following process: 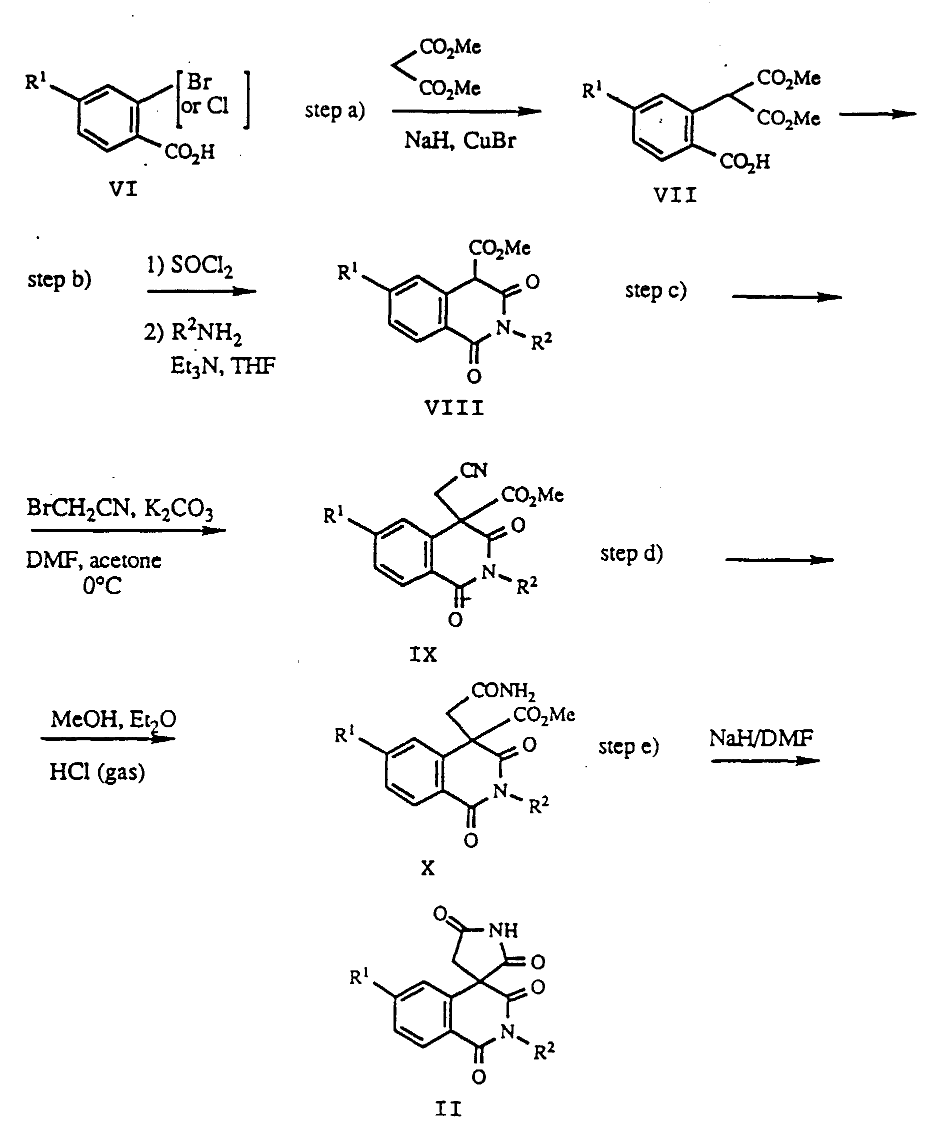 wherein R¹ and R² are as defined above.

The present invention includes the compound of formula (IX) 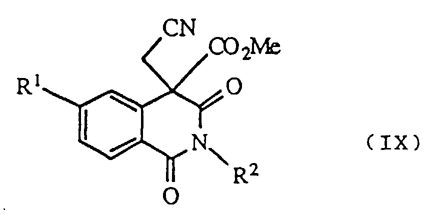 wherein R¹ and R² are as defined above.

(IX) is useful as an intermediate for preparing spiro[isoquinoline]-4(1H),3'-pyrrolidine]-1,2',3,5'(2H)-tetrones of formula (II) which have strong aldose reductase inhibiting activity and are expected useful as remedies for diabetic complications.

The following examples further illustrate this invention.

### Example 1

### 2-[(4-Bromo-2-fluorophenyl)methyl]-6-fluorospiro[isoquinoline-4(1H),3'-pyrrolidine]-1,2',3,5'(2H)-tetrone

### Step a)

### (2-Carboxy-5-fluorophenyl)propanedioic Acid Dimethyl Ester

To a rapidly stirred cold suspension (0°C) of 2-chloro-4-fluorobenzoic acid (20.0 g, 114.6 mmol), cuprous bromide (1.64 g, 11.46 mmol) and dimethyl malonate (250 g) was added NaH (80% in mineral oil, 8.25 g, 275.04 mmol) over a 30 minute period, while a stream of dry N2 was passed over the mixture. After the addition of the NaH had been completed, the mixture wa stirred at 85°C for 3 hours. At this point, the suspension had turned to a solid mass, which was dissolved in H₂O (1000 mL). The aqueous layer was extracted with diethyl ether (3 x 500 mL) and was acidified with HCl (2N). The mixture was extracted with EtOAc and dried over MgSO₄. Evaporation gave an off-white solid which was recrystallized from ether/hexane (after cooling to -20°C) to give a white solid (27.5 g, 89%). ¹H NMR (DMSO-d₆, 400 MHz): δ 3.68 [s, 6H, (-CO₂Me)2], 5.79 (s, 1H, Ar-H), 7.12 (dd, J = 10.06 Hz, 2.61 Hz, 1H, Ar-H), 7.33 (dt, J = 8.48 Hz, 2.64 Hz, 1H, Ar-H), 8.03 (dd, 8.77 Hz, 6.17 Hz, 1H, Ar-H); IR (KBr, cm⁻¹): 3400-2700 (CO₂H), 1730 (CO), 1680 (CO); MS (m/e): 270 (M⁺)-CH₃OH), 210 (M⁺-CH₃OH,-CO), 151 (M⁺-CH₃OH-CO-CO₂CH₃); M.P. 121.5-123.0°C.

| | | |
|---|---|---|
| Anal. Calc'd: | C, 53.34; | H, 4.10 |
| Found: | C, 53.36; | H, 3.93 |

The following compounds were prepared in substantially the same manner as that of Example 1 Step a):

### (2-Carboxyphenyl)propanedioic Acid Dimethyl Ester

¹H NMR (DMSO-d₆, 400 MHz): δ 3.67 [s, 6H, -CH(C)₂CH₃)₂], 5.72 [s, 1H, -CH(CO₂CH₃)₂], 7.3 (d, J = 7.76 Hz, 1H, Ar-H), 7.45 (dt, J = 7.66 Hz, 1.12 Hz, 1H, Ar-H), 7.6 (dt, J = 7.66 Hz, 1.45 Hz, 1H, Ar-H), 7.94 (dd, J = 7.8 Hz, 1.33 Hz, 1H, Ar-H), 13.2 (s, 1H, -CO₂H); IR (KBr, cm⁻¹): 3300-2700 (CO₂H), 1750 (CO), 1730 (CO), 1680 (CO); MS (m/e): 252 (M⁺), 220 (M⁺-CH₃OH), 188 (M⁺-2xCH₃OH); M.P. 119-120°C.

| | | |
|---|---|---|
| Anal. Calc'd: | C, 57.14; | H, 4.80 |
| Found: | C, 57.05; | H, 4.78 |

### (2-Carboxy-5-chlorophenyl)propanedioic Acid Dimethyl Ester

¹H NMR (DMSO-d₆, 200 MHz): 6 3.69 [s, 6H, (-CO₂Me)₂], 5.78 [s, 1H, Ar-CH(CO₂Me)₂], 7.38 (d, J = 1.8 Hz 1H, Ar-H), 7.58 (dd, J = 7.8 Hz, 1.8 Hz, 1H, Ar-H), 7.96 (d, J = 8.2 Hz, 1H, Ar-H), 13.5 (br s, 1H, -CO₂H); IR (KBr, cm⁻¹): 3200-2700 (CO₂H), 1760 (CO), 1740 (CO), 1690 (CO); MS (m/e): 286 (20 M⁺), 254 (64, M⁺-CH₃OH), 222 (60, M⁺-2xCH₃OH)

| | | |
|---|---|---|
| Anal. Calc'd: | C, 50.28; | H, 3.87 |
| Found: | C, 50.40; | H, 3.87 |

### Step b)

### 2-[(4-Bromo-2-fluorophenyl)methyl-6-fluoro 1,2,3 4-tetrahydro-1,3-dioxo-4-isoquinolinecarboxylic Acid Methyl Ester

A mixture of (2-carboxy-5-fluorophenyl)propanedioic acid dimethyl ester (6.0 g, 22.22 mmol) and SOCl₂ (50 mL) was refluxed for 1 hour. The volatiles were removed in vacuo and the acid chloride was ddissolved in THF (20 mL). In a second flask were placed 4-bromo-2-fluorobenzylamine (4.98 g, 24.44 mmol), triethylamine (15.48 mL, 111.1 mmol) and THF (150 mL). The contents of the first flask were added to the second flask and the mixture was stirred for 20 minutes. The formed suspension was poured into H₂O (1500 mL), stirred for 10 minutes and acidified with HCl (2N). The mixture was extracted with EtOAc and the organic layer was dried over MgSO₄. Evaporation gave a yellowish solid which was recrystallized from acetone/ether/hexane (after cooling -20°C) to give a white solid (7.85 g, 83%). ¹H NMR (DMSO-d₆, 400 MHz): 6 3.98 (s, 3H, -CO₂CH₃), 5.27 (s, 2H, -NCH₂-), 7.08 (t, J = 7.95 Hz, 2H, Ar-H), 7.2 (m, 1H, Ar-H), 7.34 (m, 2H, Ar-H, -OH, 7.54 (m, 1H, Ar-H), 8.1-8.26 (m, 2H, Ar-H); IR (KBr, cm⁻¹): 1680 (CO), 1660 (CO), 1610 (CO); MS (m/e): 423 (M⁺), 391(M⁺-CH₃OH); M.P.157-158°C.

| | | | |
|---|---|---|---|
| Anal. Calc'd: | C, 50.97; | H, 2.85; | N, 3.30 |
| Found: | C, 50.86; | H, 2.86; | N, 3.33 |

The following compounds were prepared in substantialiy the same manner as that of Example 1 Step b):

### 2-[(4-Bromo-2-fluorophenyl)methyl]-1,2,3,4-tetrahydro-1,3-dioxo-4-isoquinolinecarboxylic Acid Methyl Ester

¹H NMR (DMSO-d₆, 400 MHz): δ [3.67, 3.99 (s, 3H, -CO₂CH₃, tautomeric], [5.06 (q, J = 15.4 Hz), 5.29 (s) 2H, N-CH₂-, tautomeric], 5.03 (s, 1H, -CHCO₂CH₃, tautomeric), 7.07-8.44 (m, 7H, Ar-H, tautomeric); IR (KBr, cm⁻¹): 1675 (CO), 1610 (CO), 1490 795 (m); MS (m/e): 405 (M⁺), 373 (M⁺-MeOH); M.P. 149-150°C.

| | | | |
|---|---|---|---|
| Anal. Calc'd: | C, 53.22; | H, 3.23; | N, 3.45 |
| Found: | C, 52.91; | H, 3.20; | N, 3.27 |

### 6-Chloro-1,2,3,4-tetrahydro-2-methyl-1,3-dioxo-4-isoquinolinecarboxylic Acid Methyl Ester

¹H NMR (DMSO-d₆, 200 MHz): δ [3.23 (s), 3.44 (s), tautomeric, 3H, -NCH₃], [3.71 (s), 4.03 (s), tautomeric, 3H, -CO₂CH₃], 7.3-8.4 (tautomeric, Ar-H, -OH, 4H); IR (KBr, cm-¹): 3440 (OH), 1680 (CO), 1600 (CO); MS (m/e): 267 (M⁺), 235 (M⁺-OMe); M.P. 166-167°C.

| | | | |
|---|---|---|---|
| Anal. Calc'd: | C, 53.85; | H, 3.77; | N, 5.23 |
| Found: | C, 53.66; | H, 3.63; | N, 5.14 |

### 1,2,3,4-Tetrahydro-2-methyl-1,3-dioxo-4-isoquinolinecarboxylic Acid Methyl Ester

¹H NMR (DMSO-d₆, 200 MHz): δ [3.24 (s), 3.46 (s), tautomeric, 3H, -NCH₃], [3.7 (s), 4.03 (s), tautomeric, 3H, -CO₂CH₃], 7.4-8.45 (tautomeric, 4H, Ar-H); IR (KBr, cm-¹): 3400 (OH), 1670 (CO), 1600 (CO); MS (m/e): 233 (M⁺), 118 (M⁺-CO₂Me, -CONCH₃); M.P. 130-131°C.

| | | | |
|---|---|---|---|
| Anal. Calc'd: | C, 61.80; | H, 4.75; | N, 6.01 |
| Found: | C, 61.62; | H, 4.89; | N, 5.92 |

### Step c)

### 2-[(4-Bromo-2-fluorophenyl)methyl]-4-cyanomethyl-6-fluoro-1,2,3,4-tetrahydro-1,3-dioxo-4-isoquinolinecarboxylic Acid Methyl Ester

To a cold (0°C) suspension of 2-[(4-bromo-2-fluorophenyl)methyl]-6-fluoro-1,2,3,4-tetrahydro-1,3-dioxo-4-isoquinolinecarboxylic acid methylester(11.0 g, 25.94mmol), K₂CO₃ (3.58 g, 25.94 mmol), DMF (50 mL) and acetone (50 mL) was added freshly distilled BrCH₂CN(3.61 mL, 51.88 mmol) and the mixture was stirred for 10 hours at 0°C and kept in the refrigerator for 4 days. The mixture was then poured into H₂O, acidified with HCl (2N), and extracted with EtOAc. The organic extracts were dried over MgSO₄. Evaporation and purification by flash chromatography hexane/EtOAc (5/1) gave a light yellow solid (11.45 g, 95.4%). ¹H NMR (DMSO-d₆, 300 MHz): δ 3.63 (s, 3H, -CO₂CH₃), 3.73 (d, J = 16.8 Hz, 1H, -HCHCN), 3.9 (d, J = 16.8 Hz, 1H, -HCHCN), 5.14 (dd, J = 15.2 Hz, 2H, -NCH₂-), 7.16 (t, J = 8.1 Hz, 1H, Ar-H), 7.36 (dd, J = 8.1 Hz, 1.8 Hz, 1H, Ar-H), 7.57 (m, 2H, Ar-H) 7.64 (dd, J = 9.3 Hz, 2.4 Hz, 1H, Ar-H), 8.3 (dd, J = 8.7 Hz, 5.7 Hz, 1H, Ar-H); IR (KBr, cm⁻¹): 2250 (CN), 1760 (CO), 1720 (CO), 1675 (CO); MS (m/e): 463 (M+H)⁺; M.P. 127-128°C.

| | | | |
|---|---|---|---|
| Anal. Calc'd: | C, 51.86; | H, 2.83; | N, 6.05 |
| Found: | C, 51.73; | H, 3.00; | N, 5.96 |

The following compounds were prepared in substantially the same manner as that of Example 2, Step c).

### 2-[(4-Bromo-2-fluorophenyl)methyl]-4-cyanomethyl-1,2,3,4-tetrahydro-1,3-dioxo-4-isoquinolinecarboxylic Acid Methyl Ester

¹H NMR (DMSO-d₆, 400 MHz): δ 3.61 (s, 3H, -CO₂CH₃), 3.72 (d, J = 17.0 Hz, 1H, -HCHCN), 3.88 (d, J = 17.0 Hz, 1H, -HCHCN), 5.14 (dd, J = 15.2 Hz, 2H, -NCH₂-), 7.17 (t, J = 8.3 Hz, 1H, Ar-H), 7.39 (dd, J = 8.3 Hz, 1.87 Hz, 1H, Ar-H), 7.55 (dd, J = 9.7 Hz, 2.1 Hz, 1H, Ar-H), 7.68 (m, 2H, Ar-H), 7.86 (dt, J = 7.7 Hz, 1.45 Hz, 1H, Ar-H), 8.21 (dd, J = 7.64 Hz, 1.25 Hz, 1H, Ar-H); IR (KBr, cm⁻¹): 2240 (CN), 1760 (CO), 1720 (CO), 1680 (CO); MS (m/e): 444 (M⁺), 404 (M⁺-CH₂CN); M.P. 108-110°C. (95% yield).

| | | | |
|---|---|---|---|
| Anal. Calc'd: | C, 53.94; | H, 3.15; | N, 6.29 |
| Found: | C, 53.94; | H, 3.38; | N, 5.93 |

### 4-Cyanomethyl-2-methyl-1,2,3,4-tetrahydro-1,3-dioxo-4-isoquinolinecarboxylic Acid Methyl Ester

¹H NMR (DMSO-d₆, 400 MHz): δ 3.31 (s, 3H, -NCH₃), 3.65 (s, 3H, -CO₂CH₃), 3.67 (d, J = 17.0 Hz, 1H -HCHCN), 3.76 (d, J = 17.0 Hz, 1H, -HCHCN), 7.58 (dd, J = 7.9 Hz, 1.04 Hz, 1H, Ar-H), 7.69 (dt, J = 7.9 Hz, 1.04 Hz, 1H, Ar-H), 7.84 (dt, J = 7.26 Hz, 1.45 Hz, 1H, Ar-H), 8.2 (dd, J = 7.3 Hz, 1.45 Hz, 1H, Ar-H); IR (KBr, cm⁻¹): 2250 (CN), 1760 (CO), 1730 (CO), 1670 (CO); MS (m/e): 272 (M⁺), 213 (M⁺-CO₂CH₃); M.P. 120-122°C. (94.3% yield).

| | | | |
|---|---|---|---|
| Anal. Calc'd: | C, 61.76; | H, 4.44; | N, 10.29 |
| Found: | C, 61.72; | H, 4.57; | N, 10.07 |

### 6-Chloro-4-cyanomethyl-2-methyl-1,2,3,4-tetrahydro-1,3-dioxo-4-isoquinolinecarboxylic Acid Methyl Ester

¹H NMR (DMSO-d₆, 400 MHz): δ 3.3 (s, 3H, -NCH₃), 3.67 (s, 3H, -CO₂CH₃), 3.74 (d, J = 17.0 Hz, 1H, -HCHCN), 3.87 (d, J = 17.0 Hz, 1H, -HCHCN), 7.7 (m, 2H, Ar-H), 8.2 (d, J = 9.1 Hz, 1H, Ar-H); IR (KBr, cm⁻¹): 2250 (CN), 1770 (CO), 1720 (CO), 1675 (CO); MS (m/e): 306 (M⁺), 247 (M⁺-CO₂CH₃); M.P. 130-132°C. (93.8% yield).

| | | | |
|---|---|---|---|
| Anal. Calc'd: | C, 54.83; | H, 3.62; | N, 9.13 |
| Found: | C, 54.74; | H, 3.74; | N, 8.89 |

### Step d)

### 4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Methyl Ester.

Dry HCl gas was passed through a cold (0°C) suspension of 2-[(4-bromo-2-fluorophenyl)methyl]-4-cyanomethyl-6-fluoro-1,2,3,4-tetrahydro-1,3-dioxo-4-isoquinolinecarboxylic acid methyl ester (7.7 g, 16.63 mmol) in dimethyl ether (300 mL) and anhydrous MeOH (2.02 mL, 49.89 mmol). The suspension during the introduction of the HCl gas turned into a solution. The mixture was kept at room temperature for 4.5 days and then hexane (500 mL) was added. Most of the volatiles were removed in vacuo to the point that a white solid started to precipitate, and the mixture was cooled to 0°C for 5 hours. The precipitated solid was filtered, washed with hexane and dried to yield a white solid (7.56 g, 95%). ¹H NMR (DMSO-d₆, 400 MHz): δ 3.49 (d, J = 16.65 Hz, 1H, -CH₂CONH₂), 3.56 (s,3H, -CO₂CH₃), 3.59 (d, J = 16.65 Hz, 1H, -CH₂CONH₂), 5.08 (dd, J = 15.48 Hz, 2H, -NCH₂), 6.94 (s, 1H, - CONH₂),7.21 (t, J = 8.22 Hz, 1H, Ar-H), 7.30 (dd, J = 8.27 Hz, 1.64 Hz, 1H, Ar-H), 7.38-7.46 (m, 2H, Ar-H), 7.51 (s, 1H, -CONH₂), 7.54 (dd, J = 9.81 Hz, 1.83 Hz, 1H, Ar-H), 8.20 (dd, J = 8.74 Hz, 5.84 Hz, 1H, Ar-H); IR (KBr, cm-¹): 3440 (NH), 3350 (NH), 1740 (CO), 1710 (CO), 1670 (CO), 1660 (CO); MS (m/e): 481 (M+H)⁺; M.P. 202-204°C.

| | | | |
|---|---|---|---|
| Anal. Calc'd: | C, 49.92; | H, 3.14; | N, 5.82 |
| Found: | C, 49.79; | H, 3.36; | N, 5.51 |

The following compounds were obtained in substantially the same manner as that of Example 1, Step d):

### 4-(2-Amino-2-oxoethyl)-2-[(4-bromo-2-fluorophenyl)methyl]-1,2,3,4-tetrahydro-1,3-dioxo-4-isoquinolinecarboxylic Acid Methyl Ester

¹H NMR (DMSO-d₆, 400 MHz): δ 3.53 (s, 3H, -CO₂CH₃), 3.51 (q, J = 16.6 Hz, 2H, -CH₂CONH₂), 5.1 (q, J = 15.4 Hz, 2H, -NCH₂-), 6.88 (s, 1H, -CONH₂), 7.23 (t, J = 8.0 Hz, 1H, Ar-H), 7.3 (dd, J = 8.3 Hz, 1.84 Hz, 1H, Ar-H), 7.46 (d, J = 7.98Hz, 1H, Ar-H), 7.52 (s, 1H, -CONH₂), 7.54-7.60 (m, 2H, Ar-H), 7.75 (dt, J = 7.76 Hz, 1.39 Hz, 1H, Ar-H), 8.1 (dd, J = 7.87 Hz, 1.22 Hz, 1H, Ar-H); IR (KBr, cm-¹): 3450 (NH), 17340 (CO), 1720 (CO), 1670 (CO); MS (m/e): 462 (M⁺); M.P. 180-182°C. (95% yield).

| | | | |
|---|---|---|---|
| Anal. Calc'd: | C, 51.84; | H, 3.46; | N, 6.05 |
| Found: | C, 51.72; | H, 3.65; | N, 5.91 |

### 4-(2-Amino-2-oxoethyl)-6-chloro-1,2,3,4-tetrahydro-2-methyl-1,3-dioxo-4-isoquinolinecarboxylic Acid Methyl Ester

¹H NMR (DMSO-d₆, 200 MHz): δ 3.26 (s, 3H, -NCH₃), 3.51 (q, J = 17.5 Hz, 2H, -CH₂CONH₂), 3.59 (s, 3H, -CO₂CH₃), 6.85 (s, 1H, -CONH₂), 7.5 (s, 1H, -CONH₂), 7.53 (d, J = 2.0 Hz, 1H, Ar-H), 7.62 (dd, J = 8.6 Hz, 2.0 Hz, 1H, Ar-H), 8.16 (d, J = 8.0 Hz, 1H, Ar-H); IR (KBr, cm⁻¹): 3420 (NH), 1760 (CO), 1710 (CO), 1660(CO); MS (m/e): 325 (M+H)⁺; M.P.220-222°C. (93.1% yield).

| | | | |
|---|---|---|---|
| Anal. Calc'd: | C, 51.78; | H, 4.03; | N, 8.63 |
| Found: | C, 51.76; | H, 4.20; | N, 8.32 |

### 4-(2-Amino-2-oxoethyl)-1,2,3,4-tetrahydro-2-methyl-1,3-dioxo-4-isoquinolinecarboxylic Acid Methyl Ester

¹H NMR (DMSO-d₆, 200 MHz): δ 3.27 (s, 3H, -NCH₃), 3.49 (s, 2H, -CH₂CO₂H), 3.56 (s, 3H, -CO₂CH₃), 6.78 (s, 1H, -CONH₂), 7.4-7.6 (m, 3H, Ar-H, -CONH₂), 7.69 (dt, J = 7.6 Hz, 2Hz, 1H, Ar-H), 8.16 (d, J = 8.2 Hz, 1H, Ar-H); IR (KBr, cm⁻¹): 3420 (NH), 1760 (CO), 1660 (CO); MS (m/e): 291 (M+H)⁺; M.P. 229-231°C. (91.9% yield).

| | | | |
|---|---|---|---|
| Anal. Calc'd: | C, 57.93; | H, 4.86; | N, 9.65 |
| Found: | C, 57.59; | H, 4.93; | N, 9.49 |

### Step e):

### 2-[(4-Bromo-2-fluorophenyl)methyl]-6-fluorospiro[isoquinoline-4(1H),3'-pyrrolidine]-1,2',3,5'(2H)-tetrone

To a solution of 4-(2-amino-2-oxoethyl)-2-[(4-bromo-2-fluorophenyl)methyl]-6-fluoro-1,2,3,4-tetrahydro-1,3-dioxo-4-isoquinolinecarboxylic acid methyl ester (6.0 g, 12.47 mmol) in DMF (50 mL) was added portionwise NaH (80% dispersion in oil, 374.2 mg, 12.47 mmol) over a 10 minute period. After stirring for 30 minutes, the mixture was poured into H₂O, acidified with HCl (2N) and extracted with EtOAc. The organic extracts were dried over MgSO₄. Evaporation and purification by flash chromatography (hexane/EtOAc 3/1) on acid washed silica gel (5% H₃PO₄ in MeOH), yielded a white solid (4.3 g, 86%). ¹H NMR (DMSO-d₆, 400 MHz): δ 3.44 (s, 2H, CONH-), 5.05 (s, 2H, -CNH₂-), 7.14 (t, J = 8.42 Hz, 1H, Ar-H), 7.32 (dd, J = 7.58 Hz, 1.26 Hz, 1H, Ar-H), 7.48 (dt, J = 8.64 Hz, 2.1 Hz, 1H, Ar-H), 7.53 (dd, 9.89 Hz, 1.89 Hz, 1H, Ar-H), 7.75 (dd, 9.69 Hz, 2.32 Hz, 1H, Ar-H), 8.22 (dd, J = 8.84 Hz, 5.89 Hz, 1H, Ar-H), 12.0 (s, 1H, -CONHCO-); IR (KBr, cm-¹): 3400 (NH), 3260 (NH), 1735 (CO), 1680(CO); MS (m/e): 449 (M+H)⁺; M.P. 230-232°C.

| | | | |
|---|---|---|---|
| Anal. Calc'd: | C, 50.80; | H, 2.47; | N, 6.24 |
| Found: | C, 50.87; | H, 2.53; | N, 6.08 |

The following compounds were prepared in substantially the same manner as that of example 1 step e):

### 2-[(4-Bromo-2-fluorophenyl)methyl]spiro[isoquinoline-4(1H),3'-pyrrolidine]-1,2',3,5'(2H)-tetrone

¹H NMR (DMSO-d₆, 400 MHz): δ 3.47 (J = 18.24 Hz, 2H, -CH₂CONH-), 5.06 (s, 2H, -NCH₂-), 7.14 (t, J = 8.2 Hz, 1H, Ar-H), 7.33 (dd, J = 8.28 Hz, 1.71 Hz, 1H, Ar-H), 7.55 (dd, J = 9.9 Hz, 1.8 Hz, 1H, Ar-H), 7.62 (t, J = 7.6 Hz, 1H, Ar-H), 7.68 (d, J = 7.78 Hz, 1H, Ar-H), 7.78 (dt, J = 8.85 Hz, 1.12 Hz, 1H, Ar-H), 8.15 (dd, J = 7.86 Hz, 1.3 Hz, 1H, Ar-H), 12.01 (s, 1H, -CONHCO-); IR (KBr, cm⁻¹): 3450 (NH), 3250 (NH), 1730 (CO), 1680 (CO); MS (m/e): 430 (M⁺), 387 (M⁺-CONH); M.P. 112-114°C.

| | | | |
|---|---|---|---|
| Anal. Calc'd: | C,52.92; | H, 2.80; | N, 6.50 |
| Found: | C, 52.61; | H, 2.70; | N, 6.46 |

### 6-Chloro-2-methylspiro[isoquinoline-4(1H)3'-pyrrolidine]-1,2',3,5'(2H)-tetrone

¹H NMR (DMSO-d₆, 400 MHz): δ 3.22 (s, 3H, -NCH₃), 3.38 (s, 2H, CH₂CONH-), 7.66 (dd, J = 8.55 Hz, 2.02 Hz, 1H, Ar-H), 7.92 (d, J = 1.97 Hz, 1H, Ar-H), 8.13 (d, J = 8.52 Hz 1H, Ar-H), 11.99 (s, 1H, -CONHCO-); IR (KBr, cm⁻¹): 3350 (NH), 1750 (CO), 1730 (CO), 1660 (CO); MS (m/e): 293 (M+H)⁺; M.P. 213-214°C

| | | | |
|---|---|---|---|
| Anal. Calc'd: | C, 53.35; | H, 3.10; | N, 9.57 |
| Found: | C, 53.43; | H, 3.09; | N, 9.38 |

### 2-Methylspiro[isoquinoline-4(1H),3'-pyrrolidine]-1,2',3,5'(2H)-tetrone

¹H NMR (DMSO-d₆, 400 MHz): δ 3.24 (s, 3H, -N-CH₃), 3.43 (q, J = 18.38 Hz, 2H, -CH₂CONH-), 7.58-7.64 (m, 2H, Ar-H), 7.74 (dt, J = 7.64 Hz, 1.2 Hz, 1H, Ar-H), 8.15 (dd, J = 7.72 Hz, 0.94 Hz, 1H, Ar-H), 12.0 (2, 1H, -CONHCO-), IR (KBr, cm-¹): 3340 (NH), 1720 (CO), 1660 (CO); MS (m/e): 258 (M⁺); M.P. 224-225°C.

| | | | |
|---|---|---|---|
| Anal. Calc'd: | C, 60.47; | H, 3.90; | N, 10.85 |
| Found: | C, 60.27; | H, 4.03; | N, 10.82 |

## Claims (Claims for the following Contracting State(s): AT, BE, CH, DE, DK, FR, GB, GR, IT, LI, LU, NL, PT, SE)

1. A process for the preparation of a compound having the formula II 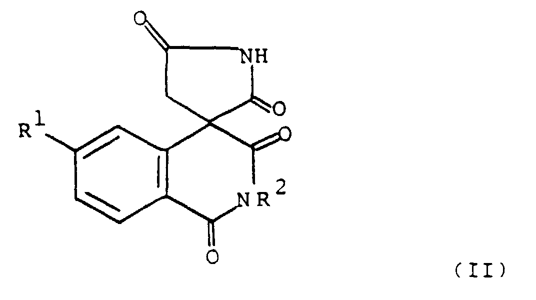 in which R¹ is hydrogen or halogen and R² is methyl or dihalogen- substituted benzyl, in which
(1) a compound having the formula III 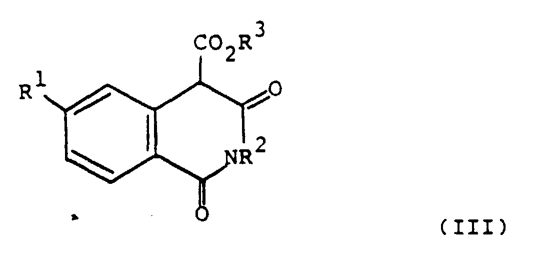 in which R¹ and R² are as defined above and -CO₂R³ is an ester group, is cyanomethylated to prepare a compound having the formula IV 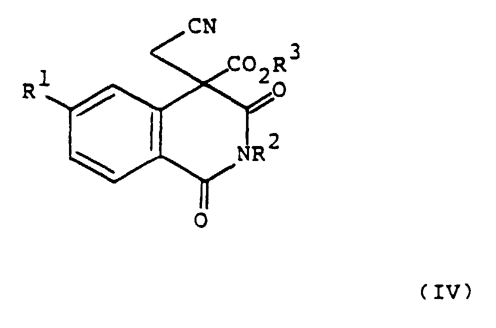 in which R¹, R² and -CO₂R³ are as defined above;
(2) the compound having formula IV is hydrated to prepare an amide having the formula V 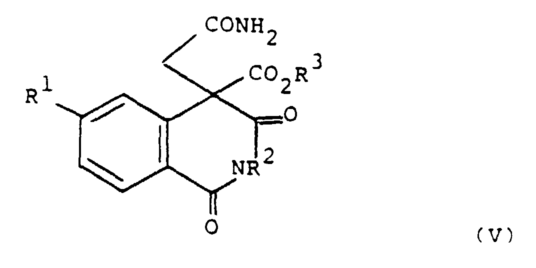 in which R¹, R² and CO₂R³ are as defined above; and
(3) the amide having the formula V is cyclized with a base to form the compound having formula II.

2. A process as claimed in claim 1 in which R¹ is halogen.

3. A process as claimed in claim 1 or 2, in which R² is dihalogen-substituted benzyl.

4. A process as claimed in claim 1, in which R¹ is fluorine and R² is (4-bromo-2-fluorophenyl) methyl.

5. A process as claimed in any one of claims 1 to 4, wherein R³ is methyl.

6. A process as claimed in any one of claims 1 to 5, wherein the compound having formula III is reacted with bromoacetonitrile in the presence of potassium carbonate.

7. A process as claimed in any one of claims 1 to 6 in which hydration of he nitrile is carried out by reaction with an alcohol and hydrogen chloride or hydrogen bromide under essentially anhydrous conditions.

8. A process as claimed in claim 7, wherein the hydration is carried out with hydrogen chloride gas and methanol.

9. A process as claimed in any one of claims 1 to 8, wherein the cyclization of the amide (V) is carried out in the presence of NaH in DMF.

10. A nitrile having the formula 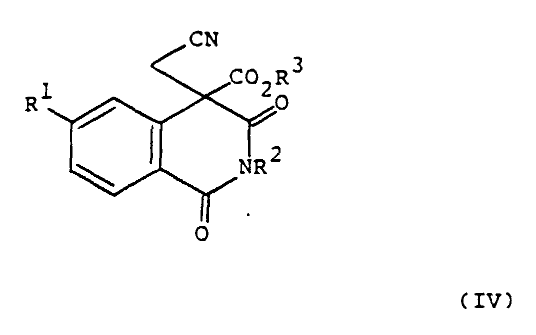 wherein R¹, R² and -CO₂R³ are as defined in claim 1.

11. A nitrile as claimed in claim 10, wherein R¹ and R² are as defined in claim 4.

12. A nitrile as claimed in claim 10 or 11 wherein R³ is methyl.

## Claims (Claims for the following Contracting State(s): ES)

1. A process for the preparation of a compound having the formula II 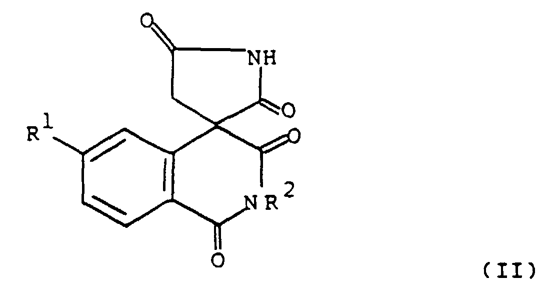 in which R¹ is hydrogen or halogen and R² is methyl or dihalogen- substituted benzyl, in which
(1) a compound having the formula III 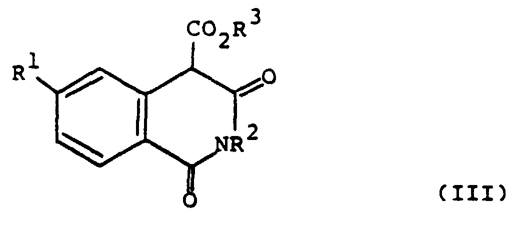 in which R¹ and R² are as defined above and -CO₂R³ is an ester group, is cyanomethylated to prepare a compound having the formula IV 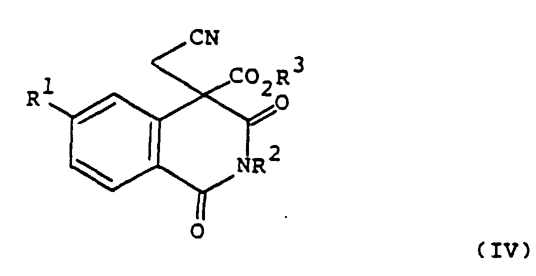 in which R¹, R² and -CO₂R³ are as defined above;
(2) the compound having formula IV is hydrated to prepare an amide having the formula V 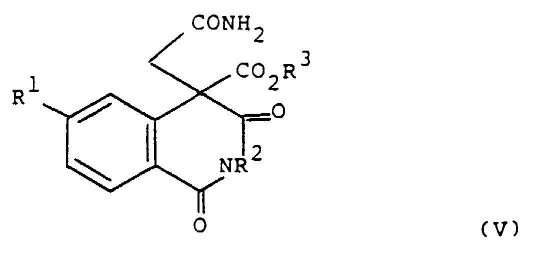 in which R¹, R² and CO₂R³ are as defined above; and
(3) the amide having the formula V is cyclized with a base to form the compound having formula II.

2. A process as claimed in claim 1 in which R¹ is halogen.

3. A process as claimed in claim 1 or 2, in which R² is dihalogen-substituted benzyl.

4. A process as claimed in claim 1, in which R¹ is fluorine and R² is (4-bromo-2-fluorophenyl) methyl.

5. A process as claimed in any one of claims 1 to 4, wherein R³ is methyl.

6. A process as claimed in any one of claims 1 to 5, wherein the compound having formula III is reacted with bromoacetonitrile in the presence of potassium carbonate.

7. A process as claimed in any one of claims 1 to 6 in which hydration of the nitrile is carried out by reaction with an alcohol and hydrogen chloride or hydrogen bromide under essentially anhydrous conditions.

8. A process as claimed in claim 7, wherein the hydration is carried out with hydrogen chloride gas and methanol.

9. A process as claimed in any one of claims 1 to 8, wherein the cyclization of the amide (V) is carried out in the presence of NaH in DMF.

10. A process for the preparation of a nitrile having formula 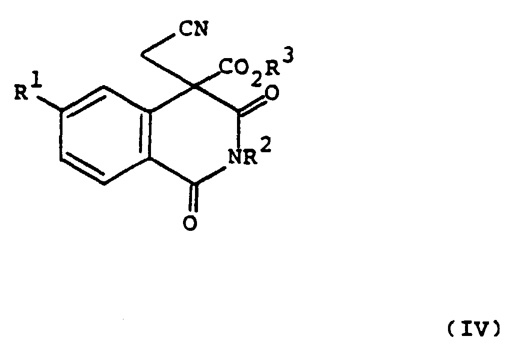 where R¹, R² and - CO₂R³ are as defined in claim 1, which comprises cyanomethylation of a compound having formula III 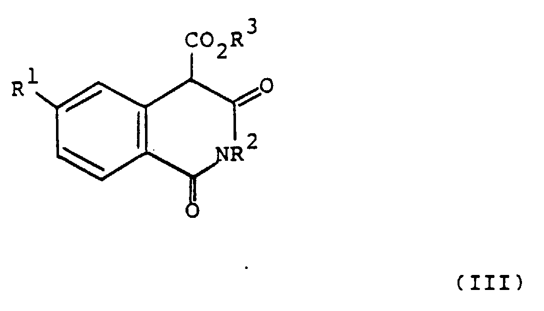 where R¹, R² and - CO₂R³ are as defined above.

11. A process as claimed in claim 10, wherein R¹ and R² are as defined in claim 4.

12. A process as claimed in claim 10 or 11, wherein R³ is methyl.

## Patentansprüche (Patentansprüche für folgende(n) Vertragsstaat(en): AT, BE, CH, DE, DK, FR, GB, GR, IT, LI, LU, NL, PT, SE)

1. Verfahren zur Herstellung einer Verbindung der Formel (II) 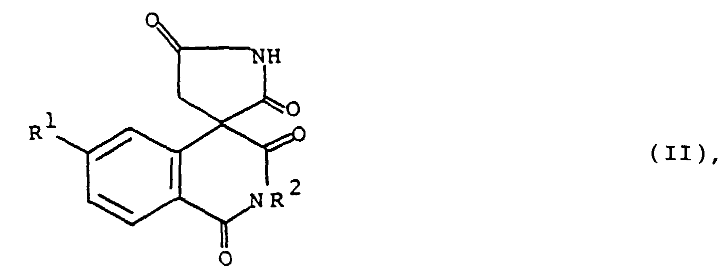 worin R¹ Wasserstoff oder Halogen bedeutet, und R² Methyl oder Dihalogen-substituiertes Benzyl darstellt, bei welchem Verfahren
(1) eine Verbindung der Formel (III) 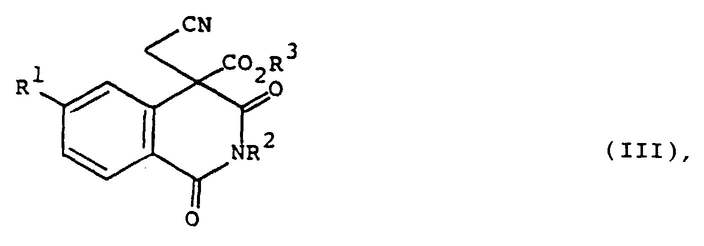 worin R¹ und R² wie oben definiert sind, und -CO₂R³ eine Ester-Gruppe bedeutet, cyanomethyliert wird, wobei eine Verbindung der Formel (IV) 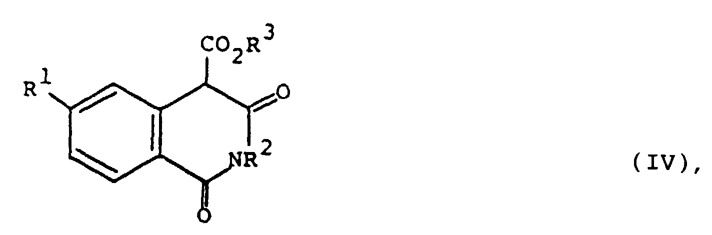 worin R¹, R² und -CO₂R³ wie oben definiert sind, hergestellt wird;
(2) die Verbindung der Formel (IV) hydratisiert wird, wobei ein Amid der Formel (V) 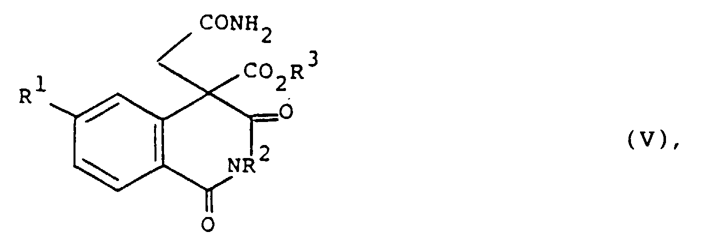 worin R¹, R² und -CO₂R³ wie oben definiert sind, hergestellt wird; und
(3) das Amid der Formel (V) mit einer Base cyclisiert wird, wobei die Verbindung der Formel (II) gebildet wird.

2. Verfahren nach Anspruch 1, wobei R¹ Halogen bedeutet.

3. Verfahren nach Anspruch 1 oder 2, worin R² Dihalogen-substituiertes Benzyl darstellt.

4. Verfahren nach Anspruch 1, wobei R¹ Fluor ist, und R² (4-Brom-2-fluorphenyl)-methyl bedeutet.

5. Verfahren nach einem der Ansprüche 1 bis 4, wobei R³ Methyl darstellt.

6. Verfahren nach einem der Ansprüche 1 bis 5, bei welchem die Verbindung der Formel (III) mit Bromacetonitril in Anwesenheit von Kaliumcarbonat umgesetzt wird.

7. Verfahren nach einem der Ansprüche 1 bis 6, bei welchem die Hydratisierung des Nitrils durch das Umsetzen mit einem Alkohol und Chlorwasserstoff oder Bromwasserstoff unter im wesentlichen wasserfreien Bedingungen durchgeführt wird.

8. Verfahren nach Anspruch 7, bei welchem die Hydratisierung mit Chlorwasserstoffgas und Methanol durchgeführt wird.

9. Verfahren nach einem der Ansprüche 1 bis 8, bei welchem die Cyclisierung des Amids (V) in Anwesenheit von NaH in DMF durchgeführt wird.

10. Nitril der Formel 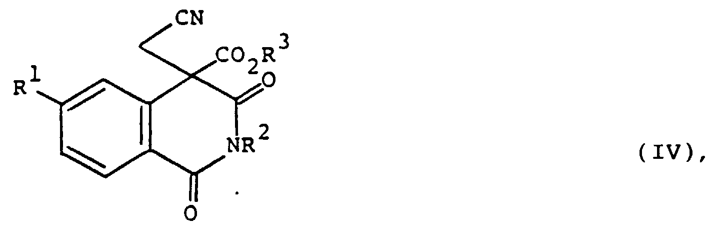 worin R¹, R² und -CO₂R³ wie in Anspruch 1 definiert sind.

11. Nitril nach Anspruch 10, worin R¹ und R² wie in Anspruch 4 definiert sind.

12. Nitril nach Anspruch 10 oder 11, worin R³ Methyl bedeutet.

## Patentansprüche (Patentansprüche für folgende(n) Vertragsstaat(en): ES)

1. Verfahren zur Herstellung einer Verbindung der Formel (II) 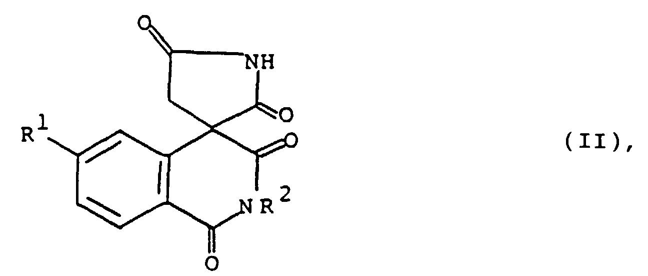 worin R¹ Wasserstoff oder Halogen bedeutet, und R² Methyl oder Dihalogen-substituiertes Benzyl darstellt, bei welchem Verfahren
(1) eine Verbindung der Formel (III) 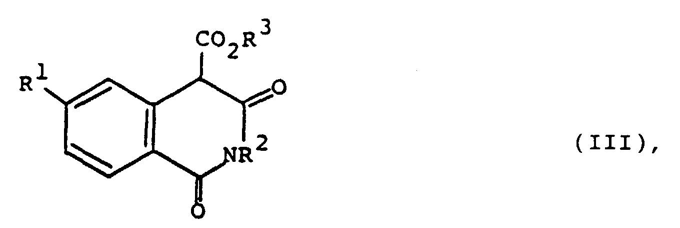 worin R¹ und R² wie oben definiert sind, und -CO₂R³ eine Ester-Gruppe bedeutet, cyanomethyliert wird, wobei eine Verbindung der Formel (IV) 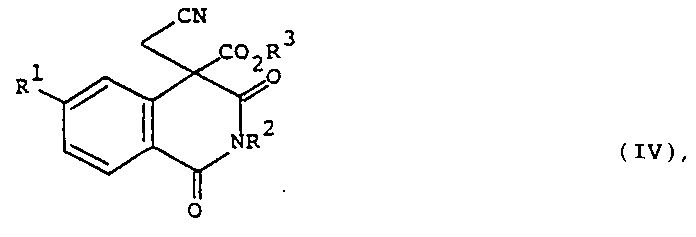 worin R¹, R² und -CO₂R³ wie oben definiert sind, hergestellt wird;
(2) die Verbindung der Formel (IV) hydratisiert wird, wobei ein Amid der Formel (V) 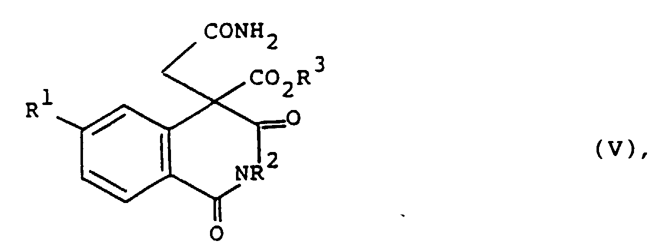 worin R¹, R² und -CO₂R³ wie oben definiert sind, hergestellt wird; und
(3) das Amid der Formel (V) mit einer Base cyclisiert wird, wobei die Verbindung der Formel (II) gebildet wird.

2. Verfahren nach Anspruch 1, wobei R¹ Halogen bedeutet.

3. Verfahren nach Anspruch 1 oder 2, worin R² Dihalogen-substituiertes Benzyl darstellt.

4. Verfahren nach Anspruch 1, wobei R¹ Fluor ist, und R² (4-Brom-2-fluorphenyl)-methyl bedeutet.

5. Verfahren nach einem der Ansprüche 1 bis 4, wobei R³ Methyl darstellt.

6. Verfahren nach einem der Ansprüche 1 bis 5, bei welchem die Verbindung der Formel (III) mit Bromacetonitril in Anwesenheit von Kaliumcarbonat umgesetzt wird.

7. Verfahren nach einem der Ansprüche 1 bis 6, bei welchem die Hydratisierung des Nitrils durch das Umsetzen mit einem Alkohol und Chlorwasserstoff oder Bromwasserstoff unter im wesentlichen wasserfreien Bedingungen durchgeführt wird.

8. Verfahren nach Anspruch 7, bei welchem die Hydratisierung mit Chlorwasserstoffgas und Methanol durchgeführt wird.

9. Verfahren nach einem der Ansprüche 1 bis 8, bei welchem die Cyclisierung des Amids (V) in Anwesenheit von NaH in DMF durchgeführt wird.

10. Verfahren zur Herstellung eines Nitrils der Formel 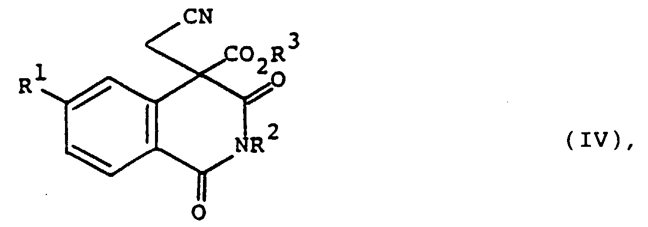 worin R¹, R² und -CO₂R³ wie in Anspruch 1 definiert sind, welches Verfahren die Cyanomethylierung einer Verbindung der Formel (III) 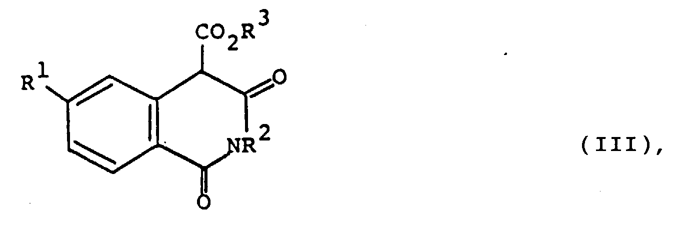 worin R¹, R² und -CO₂R³ wie oben definiert sind, umfaßt.

11. Verfahren nach Anspruch 10, wobei R¹ und R² wie in Anspruch 4 definiert sind.

12. Verfahren nach Anspruch 10 oder 11, wobei R³ Methyl bedeutet.

## Revendications (Revendications pour l'(les) Etat(s) contractant(s) suivant(s): AT, BE, CH, DE, DK, FR, GB, GR, IT, LI, LU, NL, PT, SE)

1. Procédé de préparation d'un composé de formule II 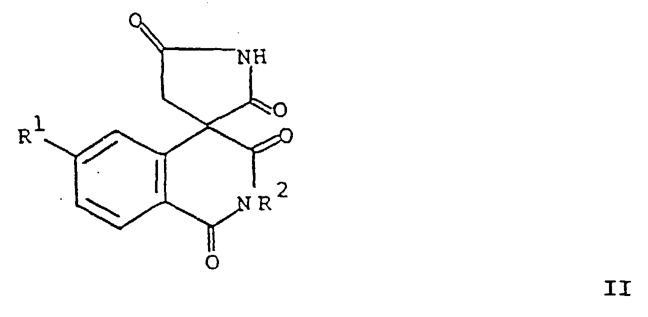 dans laquelle R¹ est hydrogène ou halogène et R² est méthyle ou un benzyle dihalogéno-substitué, dans lequel
(1) un composé de formule III 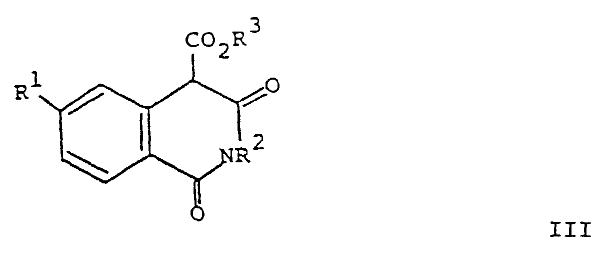 dans laquelle R¹ et R² sont tels que définis ci-dessus et -CO₂R³ est un radical ester, est cyanométhylé pour préparer un composé de formule IV 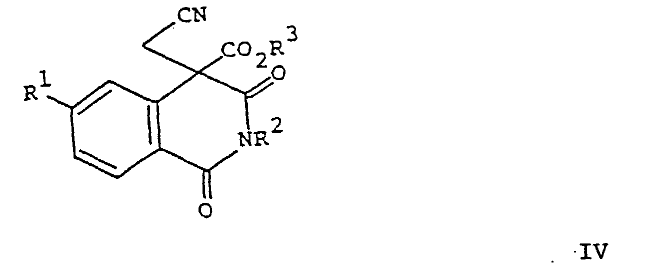 dans laquelle R¹, R² et -CO₂R³ sont tels que définis ci-dessus;
(2) le composé de formule IV est hydraté pour préparer un amide de formule V 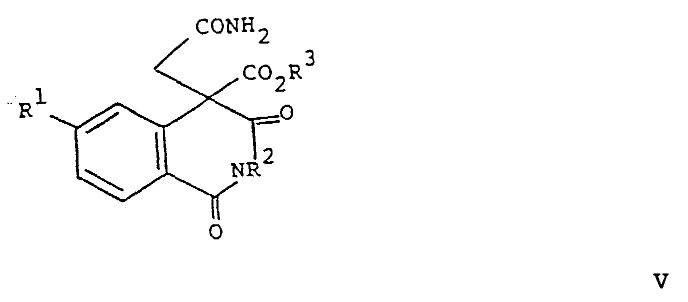 dans laquelle R¹, R² et -CO₂R³ sont tels que définis ci-dessus; et
(3) l'amide de formule V est cyclisé avec une base pour former le composé de formule II.

2. Procédé selon la revendication 1, dans lequel R¹ est halogène.

3. Procédé selon la revendication 1 ou 2, dans lequel R² est un benzyle dihalogéno-substitué.

4. Procédé selon la revendication 1, dans lequel R¹ est fluor et R² est (4-bromo)-2-fluorophényl)méthyle.

5. Procédé selon l'une quelconque des revendications 1 à 4, dans lequel R³ est méthyle.

6. Procédé selon l'une quelconque des revendications 1 à 5, dans lequel le composé de formule III est mis à réagir avec du bromoacétonitrile en présence de carbonate de potassium.

7. Procédé selon l'une quelconque des revendications 1 à 6, dans lequel l'hydratation du nitrile est effectuée par réaction avec un alcool et du chlorure d'hydrogène ou du bromure d'hydrogène sous des conditions essentiellement anhydres.

8. Procédé selon la revendication 7, dans lequel l'hydratation est effectuée avec du chlorure d'hydrogène gazeux et du méthanol.

9. Procédé selon l'une quelconque des revendications 1 à 8, dans lequel la cyclisation de l'amide V est effectuée en présence de NaH dans du DMF.

10. Nitrile de formule 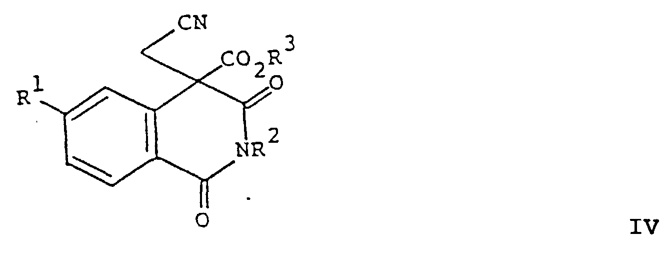 dans lequel R¹, R² et -CO₂R³ sont tels que définis dans la revendication 1.

11. Nitrile selon la revendication 10, dans lequel R¹ et R² sont tels que définis dans la revendication 4.

12. Nitrile selon la revendication 10 ou 11, dans lequel R³ est méthyle.

## Revendications (Revendications pour l'(les) Etat(s) contractant(s) suivant(s): ES)

1. Procédé de préparation d'un composé de formule II 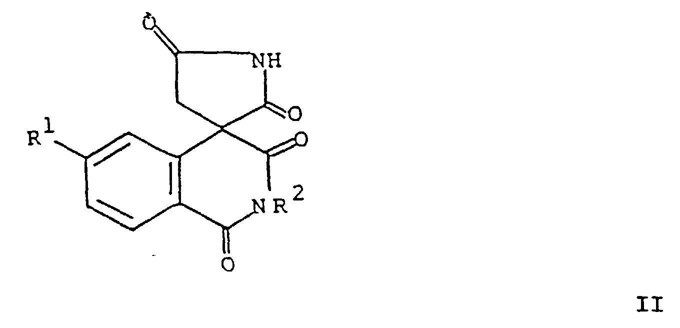 dans laquelle R¹ est hydrogène ou halogène et R² est méthyle ou benzyle dihalogéno-substitué, dans lequel
(1) un composé de formule III 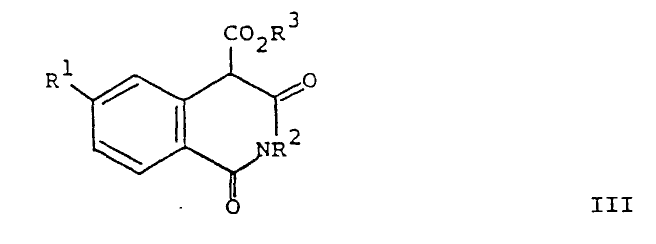 un composé de formule IV 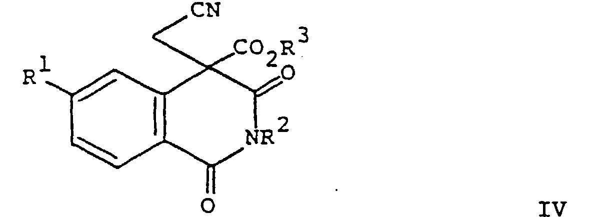 dans laquelle R¹, R² et -CO₂R³ sont tels que définis ci-dessus;
(2) le composé de formule IV est hydraté pour préparer un amide ayant la formule V 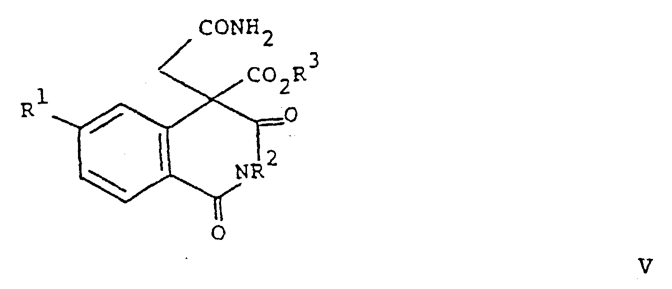 dans laquelle R¹, R² et -CO₂R³ sont tels que définis ci-dessus; et
(3) l'amide de formule V est cyclisé avec une base pour former le composé de formule II.

2. Procédé selon la revendication 1, dans lequel R¹ est halogène.

3. Procédé selon la revendication 1 ou 2, dans lequel R² est un benzyle dihalogéno-substitué.

4. Procédé selon la revendication 1, dans lequel R¹ est le fluor et R² est un (4-bromo)-2-fluorophényl)méthyle.

5. Procédé selon l'une quelconque des revendications 1 à 4, dans lequel R³ est méthyle.

6. Procédé selon l'une quelconque des revendications 1 à 5, dans lequel le composé de formule III est mis à réagir avec du bromoacétonitrile en présence de carbonate de potassium.

7. Procédé selon l'une quelconque des revendications 1 à 6, dans lequel l'hydratation du nitrile est effectuée par réaction avec un alcool et du chlorure d'hydrogène ou du bromure d'hydrogène sous des conditions essentiellement anhydres.

8. Procédé selon la revendication 7, dans lequel l'hydratation est effectuée avec du chlorure d'hydrogène gazeux et du méthanol.

9. Procédé selon l'une quelconque des revendications 1 à 8, dans lequel la cyclisation de l'amide V est effectuée en présence de

10. Procédé de préparation d'un nitrile de formule 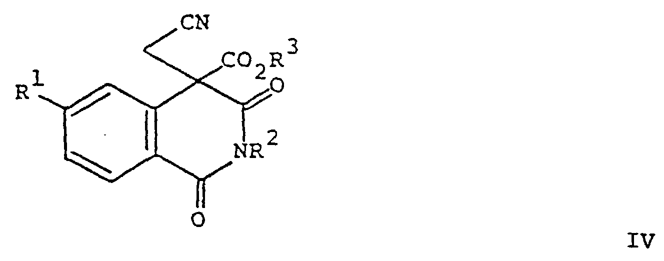 dans laquelle R¹, R² et -CO₂R³ sont tels que définis dans la revendication 1, qui comprend la cyanométhylation d'un composé de formule III 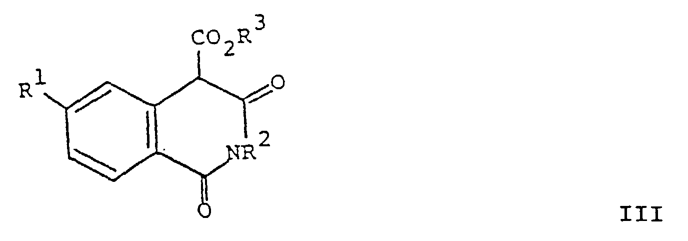 dans laquelle R¹, R² et -CO₂R³ sont tels que définis ci-dessus.

11. Procédé selon la revendication 10, dans lequel R¹ et R² sont tels que définis dans la revendication 4.

12. Procédé selon la revendication 10 ou 11, dans lequel R³ est méthyle.
